# EUROPÄISCHE PATENTANMELDUNG

(11) **EP 1 415 596 A1**
(43) Veröffentlichungstag der Anmeldung: **06.05.2004**
(21) Anmeldenummer: 02405916.4
(22) Anmeldetag: 28.10.2002
(51) Int. Cl.: A61B 8/00

(54) **Druckmesseinrichtung für ultraschallmessvorrichtungen**

(71) Anmelder: Baumann, Ulrich André, 3110 Münsingen (CH)
(72) Erfinder: Baumann, Ulrich André, 3110 Münsingen (CH)
(74) Vertreter: Störzbach, Michael Andreas

(57) **Zusammenfassung**

Eine Druckmesseinrichtung (5) zum Ankoppeln an einen Ultraschallmesskopf (7) umfasst ein Gehäuse mit einem rückwärtigen Fenster aus einem steifen, Ultraschall-durchlässigen Material, z.B. PTFE in einer Dicke von höchstens 1 mm, und eine vorderseitige Öffnung, die mit einer Membran aus einem Silikonelastomer, bevorzugt mit einer Dicke von 0,4 bis 0,5 mm, verschlossen ist. Der Innenraum ist mit einer Ultraschall-durchlässigen Flüssigkeit, insbesondere einem Glyzerin-Wassergemisch, gefüllt. Mit der Druckmesseinrichtung können die bekannten Ultraschallmessungen an Lebewesen unter Messung des Auflagedrucks durchgeführt werden.

## Beschreibung

Die vorliegende Erfindung bezieht sich auf eine Druckmesseinrichtung zur Durchführung von Ultraschallmessungen an Lebewesen, insbesondere Menschen, gemäss Oberbegriff des Anspruchs 1. Des Weiteren bezieht sie sich auf eine Ultraschall-Messvorrichtung mit einer solchen Einrichtung und Verwendungen der Druckmesseinrichtung und der Ultraschallmessvorrichtung.

Bei diagnostischen Ultraschalluntersuchungen wird ein kommerzieller Schallkopf mittels eines Kontaktgels auf die zu untersuchende Person aufgelegt, z.B. die Haut der Bauchdecke. Durch kommerziell erhältliche Apparaturen wird aus dem im Schallkopf ausgesendeten und durch Reflexion wieder empfangenen Signal ein Bild erzeugt, das z.B. in der Medizin die Darstellung von tiefer liegenden und dem Auge verborgenen Organen und Geweben erlaubt.

Die Anwendung eines Druckes auf den Schallkopf zur Charakterisierung von druckbedingten Gewebe- und Gefässveränderungen ist gut etabliert (z.B. Venenkompressionen zur Thrombosediagnostik). Der Druck auf den Ultraschallmesskopf und damit die Auflagefläche wurde dabei jedoch rein gefühlsmässig durch den Anwender aufgebracht. Der bei der Ultraschalluntersuchung in der Medizin ausgeübte Druck auf das zu untersuchende Gewebe/Organsystem ist offensichtlich von Untersucher zu Untersucher verschieden und abhängig von seinem Ausbildungsstand und seiner Erfahrung.

Zur besseren Darstellung von Gefässstrukturen werden in der Medizin auch Dopplerverfahren eingesetzt, die eine teure Zusatzausrüstung zu den kommerziellen Ultraschallgeräten bedingen.

Eine solche Anordnung ist aus der Patentschrift US-A-6 086 533 bekannt. Darin wird einem Ultraschallmesskopf eine Blase vorgesetzt. Die Blase ist mit einer akustisch durchlässigen Flüssigkeit gefüllt. Die ganze Anordnung ist in ein zylindrisches Gehäuse eingebaut, an dessen einem offenen Ende die Blase zugänglich ist. Wird die Anordnung auf die Haut gesetzt, so kann der Anpressdruck durch Messen des Innendrucks der Blase bestimmt werden, und es kann ein Ultraschall-Signal durch die Blase in das Gewebe eingestrahlt und das Echo ausgewertet werden.

Praktische Versuche mit derartigen Anordnungen zeigten jedoch, dass die räumliche Auflösung der in der Medizin üblichen Ultraschall-Messköpfe durch das Vorschalten einer derartigen Druckmesseinrichtung regelmässig stark beeinträchtigt bis unbrauchbar wird.

Eine Aufgabe der vorliegenden Erfindung besteht darin, eine Druckmesseinrichtung für einen Ultraschallmesskopf anzugeben, die die Messeigenschaften des Ultraschallmesskopfes weniger beeinträchtigt.

Eine solche Einrichtung ist im Anspruch 1 angegeben. Die weiteren Ansprüche geben bevorzugte Ausbildungen, Ultraschall-Messvorrichtungen damit und Anwendungen an.

Demgemäss besteht die Druckmesseinrichtung im Wesentlichen aus einem starren Gehäuse, z.B. in Form eines flachen Hohlzylinders. An der Rückseite ist eine steife Membran eingelassen, die der Ankoppelung an den Ultraschallmesskopf dient. Die Vorderseite ist mit einer flexiblen Membran verschlossen. Der Innenraum ist mit einer Ultraschalldurchlässigen Flüssigkeit möglichst vollständig, idealerweise blasenfrei, gefüllt.

Es hat sich gezeigt, dass insbesondere die flexible Membran, die an die Körperoberfläche des Patienten angelegt wird, aber auch die steife Membran einen starken Einfluss auf die Qualität der Ultraschall-Bilder und insbesondere deren räumliche bzw. zweidimensionale Auflösung haben.

Bei der steifen Membran ist insbesondere eine genügend kleine Dicke zu wählen.

Für die flexible Membran wurde als einziges anwendbares Material Silikon ermittelt.

Die Flüssigkeit muss generell Ultraschall-durchlässig sein. Bewährt haben sich allgemein wasserhaltige Flüssigkeiten und insbesondere Glycerin-Wassermischungen. Auch die vom Ultraschall zu durchwandernden Flüssigkeitsschichten sollten möglichst dünn gehalten werden.

Eine erste Anwendung findet die erfindungsgemässe Druckmesseinrichtung in der Untersuchung druckabhängiger Veränderungen im Gewebe eines Patienten, wobei der untersuchenden Person neben den Druckdaten auch die üblichen Ultraschall-Bilder und daraus abgeleitete Informationen zur Verfügung stehen.

Eine weitere Anwendung findet die erfindungsgemässe Vorrichtung in der Ausbildung: Die Ultraschalltechnik des einzelnen Untersuchers ist bekanntlich individuell verschieden. Mit der Vorrichtung können in der Ausbildung oder bei Resultatvergleichen diese unterschiedlichen Untersuchungstechniken/Auflagedrücke des Schallkopfes harmonisiert werden und/oder Auszubildende können von Anfang an den richtigen Schallkopfauflagedruck erlernen.

Im Weiteren ist es denkbar, dass durch einfache quantifizierbare Aussagen mit der vorgestellten Erfindung teure Zusatzuntersuchungen wie Farbduplex nicht mehr notwendig sind oder nur noch gezielt eingesetzt werden müssen.

Die Erfindung soll weiter an Ausführungsbeispielen unter Bezugnahme auf Figuren erläutert werden. Es zeigen:

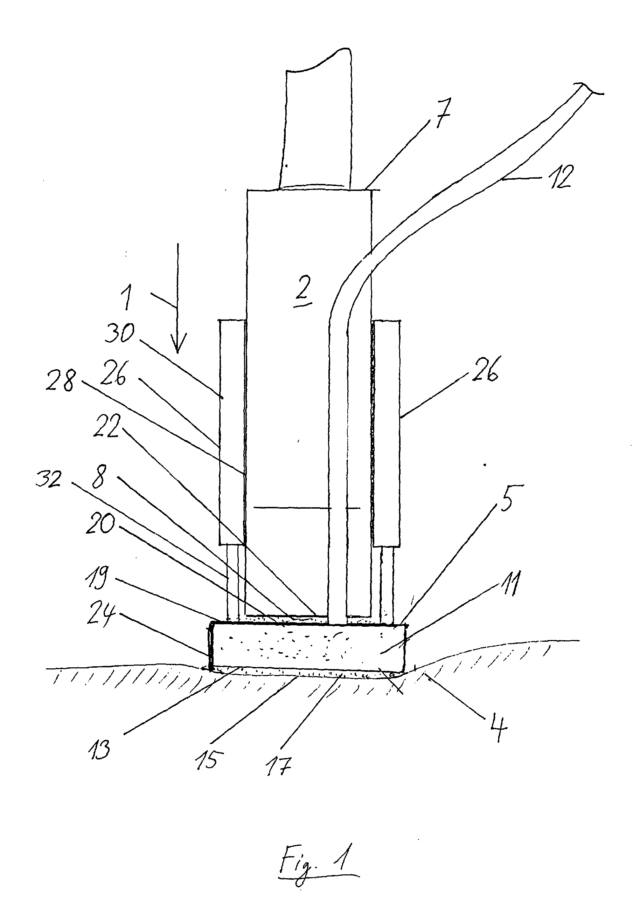

- Fig. 1:: Schematisch einen Ultraschallmesskopf mit Druckmesseinrichtung in Seitenansicht;
- Fig. 2:: eine Ansicht von hinten auf die Druckmesseinrichtung;
- Fig. 3:: einen Schnitt gemäss III-III in Fig. 2; und
- Fig. 4:: eine Ultraschall-Aufnahme eines Ultraschall-Messkopfes mit erfindungsgemässer Druckmesseinrichtung.

Die beispielhafte erfindungsgemässe Ausführung zeichnet sich dadurch aus, dass der Auflagedruck, d.h. die gerichtete Kraft 1 des Ultraschallkopfes 2 auf das Gewebe 4 (z.B. die Haut einer Person), während der Untersuchung quantitativ gemessen und dargestellt wird. Zu diesem Zweck wird eine schalldurchlässige Druckmesseinrichtung in Form einer Messkapsel 5 an einen kommerziell erhältlichen Schallkopf 7 angekoppelt. Denkbar ist auch, dass die Druckmesseinrichtung in den Schallkopf integriert ausgeführt ist. Die Grösse und Form der Messkapsel 5 kann je nach Anwendung und Aufgabe variiert werden. Neben der fixen Kopplung oder konstruktiven Integration an oder in den Schallkopf sind verschiedene Ankoppelungen an bekannte Schallköpfe denkbar. In der Regel wird zwischen der Druckmesseinrichtung und dem Ultraschallkopf ebenfalls ein Gel 8 angewendet, um die Kopplung zu verbessern.

Im Rahmen der Erfindung sind verschiedene messtechnische Lösungen möglich, wie eine Druckmessung durch kommerzielle Druckwandler in der zwischen Schallkopf und Auflagemembran befindlichen Flüssigkeit, Druckveränderung der Auflagemembran etc.

Im Ausführungsbeispiel ist die Flüssigkeit 11 im Inneren der Messkapsel 5 für die Druckmessung über eine Leitung 12 mit einem Druckmessgerät (nicht dargestellt) verbunden. Geeignete Druckmessgeräte sind an sich bekannt und daher nicht beschrieben. Die Flüssigkeit muss für Ultraschall idealerweise vollständig transparent sein. Bewährt hat sich eine Glycerin-Wassermischung im Massenverhältnis 85:15.

Die Messkapsel 5 ist durch eine flexible, Ultraschall-durchlässige Membran 13 an der Seite abgeschlossen, die zur Auflage auf das Gewebe 4 dient.

In praktischen Versuchen hat sich gezeigt, dass das Material und die Auslegung dieses Membrans einen grossen Einfluss auf die Qualität der Ultraschall-Messung hat. In zahlreichen Versuchen wurde Silikonelastomer als geeignet ermittelt, bevorzugt der Typ MVQ (internat. Kurzzeichen), der sich durch folgende Daten auszeichnet:
- Härte: 50° Shore A
- Dichte: 1,15 g/cm³
- Aussehen: milchig transparent

Die Dicke der Membran sollte ebenfalls möglichst gering sein, z.B. 0,4 bis 0,5 mm, das Material MVQ konnte problemlos mit einer Dicke von 1 mm eingesetzt werden. Kleinere Dicken wären von Vorteil, sind jedoch leichter zu beschädigen. Dickere Membranen, insbesondere dicker als 3 mm, beeinträchtigen dagegen das Ultraschall-Messergebnis 13 zu stark.

Zwischen der Membran und der Gewebeoberfläche 15 wird bei der Messung wie üblich ebenfalls ein Gel 17 angewendet, um die Utraschall-Kopplung zu verbessern.

In die Rückseite 19 der Messkapsel 5 ist ein Fenster 20 eingelassen. Das Fenster 20 besteht aus einem Ultraschall durchlässigen Material, das bevorzugt eine geringe Biegsamkeit aufweist. Beim Aufsetzen des Ultraschall-Kopfes 7 auf die Rückseite 19 kann sich damit die Membran etwas an die Form der Stirn 22 des Messkopfes anpassen, die oft mehr oder weniger abgerundet ist. Für das Fenster hat sich Teflon® (Polyfluorethylen, Du Pont) in einer Dicke von 0,5 bis 0,7 mm als geeignet erwiesen. Ab 1 mm Dicke wurde eine deutliche Verschlechterung der Qualität der Ultraschall-Messung beobachtet, ab 3 mm Dicke waren die Messungen praktisch unbrauchbar.

Für eine bessere Anpassung an die Stirn 22 des Messkopfes 7 kann das Fenster auch gekrümmt ausgeführt werden wie in Fig. 3 dargestellt.

Das Fenster 20 und die Membran 13 sind in einem Rahmen 24 aus Metall gehalten. Grundsätzlich gilt, dass sämtliche Gehäusebestandteile der Messkapsel 5 ausser der Membran 13 so starr als möglich ausgeführt sind. Im Hinblick auf den Rahmen 24, der keine besonderen Eigenschaften hinsichtlich Ultraschall aufweisen muss, kann also auch an andere Materialien, wie z.B. harte Kunststoffe, ggf. mit Einlagen, gedacht werden.

An der Rückseite der Messkapsel 5 sind am Rahmen Halter 26 befestigt, zwischen die der Messkopf 7 eingeschoben ist. Die Halter 26 können z.B. mit Federelementen (nicht dargestellt) ausgestattet sein, um den Messkopf 7 zu halten. Dargestellt ist eine Variante mit Klettverschlüssen 28, die eine stabile, aber druckfreie Fixierung des Messkopfes 7 auf der Messkapsel 5 gestatten. Die Halter 26 sind dazu als Flügel 30 ausgebildet, die um die Stäbe 32 schwenkbar sind und an ihren Seiten ein Klettband tragen.

Für die Ausführung der Verbindung zwischen Messkapsel 5 und Messkopf 7 sind eine Vielzahl Varianten denkbar, im Extremfall auch die Ausführung als untrennbare Verbindung, d.h. als ein Ultraschall-Messkopf mit Druckmesseinrichtung.

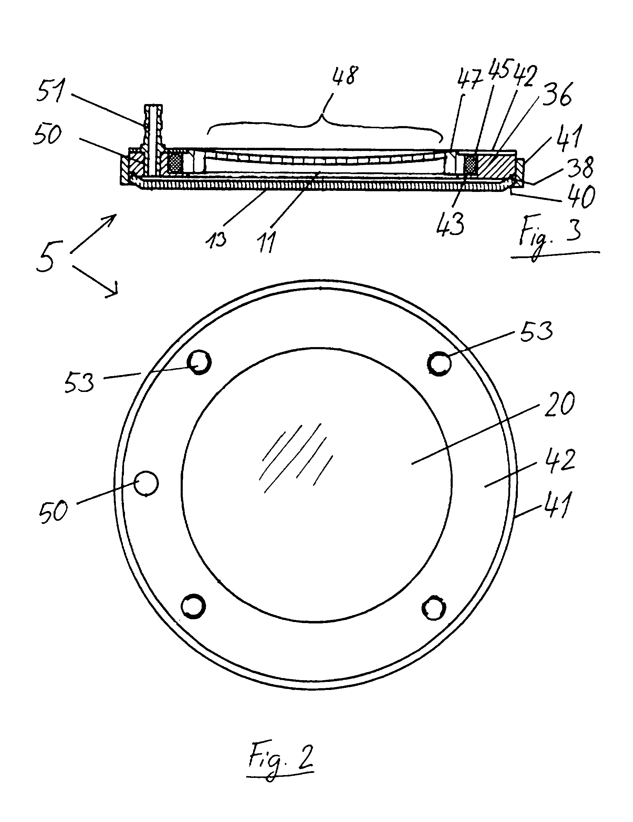

Figg. 2 und 3 zeigen eine konkrete Konstruktion der Messkapsel 5. Grundelement ist ein Ring 36 mit rechteckigem Querschnitt. Aussen unten ist eine Kante abgeschrägt, um eine Auflagefläche 38 um die flexible Membran 13 zu bilden. Die Membran 13 wird am Umfang von einem Vorsprung 40 eines ersten Klemmrings 41 auf die Auflagefläche 38 gedrückt und damit befestigt. Der Klemmring 41 kann allein auf den Ring 36 aufgeklemmt sein, denkbar ist aber auch zusätzlich oder alternativ eine andere Befestigung, wie Kleben oder Schrauben.

Auf der Oberseite liegt auf dem Ring 36 ein zweiter, flacher Klemmring 42 auf. Zwischen dem Klemmring 42 und einer Stufe 43 an der Unterseite des Rings 36 wird das Fenster 20 eingeklemmt. Zur Abdichtung ist um das Fenster 20 ein O-Ring 45 gelegt.

Der von der Flüssigkeit 11 gefüllte Hohlraum weist eine Höhe von etwa 1 mm auf. Dieser Wert ist jedoch nicht kritisch.

Das Fenster 20 weist am Umfang 47 eine Dicke entsprechend dem Abstand zwischen Klemmring 42 und Vorsprung 43 auf. Die Mittelzone 48 weist dagegen eine geringe Dicke wie oben beschrieben auf, z.B. 0,5 - 0,7 mm, um den Ultraschalldurchgang möglichst wenig zu behindern.

Dargestellt ist eine gekrümmte Ausführung als Anpassung an einen Ultraschallkopf mit gekrümmter Stirn.

Durch den Ring 36 verläuft eine Bohrung 50, in die der Anschlussnippel 51 für die Leitung 12 zum Druckmessgerät eingesetzt ist. Weitere Sacklöcher 53 dienen der Anbringung der Halter 26 und der Befestigung des Klemmrings 42 auf dem Ring 36.

Dargestellt ist eine scheibenförmige Ausführung, denkbar sind jedoch auch andere Formen wie z.B. rechteckig in Anpassung an die oft rechteckige Stirn von Ultraschallmessköpfen.

Der Innenraum 55 wird möglichst blasenfrei mit der Ultraschall-transparenten Flüssigkeit gefüllt. Z.B. kann dazu Vakuum über den Nippel 51 angelegt werden. Danach lässt man die Flüssigkeit selbsttätig einfliessen, die dann den Innenraum vollständig ausfüllt.

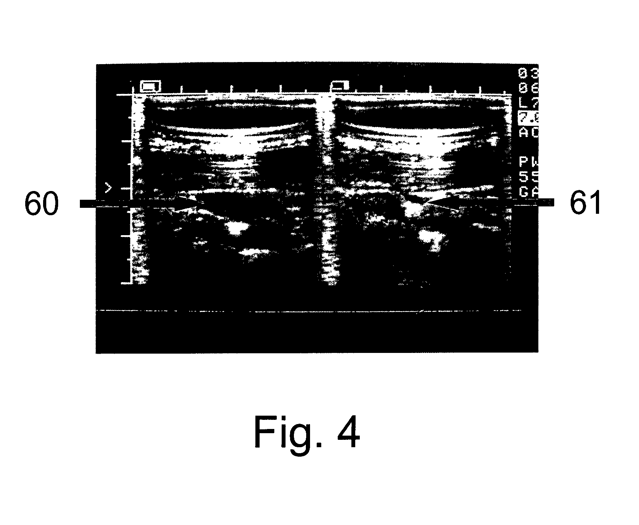

Eine Messung mit einem Ultraschall-Messkopf 7 mit vorgeschalteter Messkapsel 5 wird im Wesentlichen wie eine normale Ultraschallmessung durchgeführt. Fig. 4 zeigt ein so erhältliches Ultraschallbild. Die Pfeile 60, 61 deuten auf die Vena jugularis interna, wobei auf der in Fig. 4 linken Abbildung kein Druck, rechts dagegen Druck ausgeübt wird. Deutlich sind die Venenwände im normalen (Pfeil 60) und im zusammengedrückten Zustand (Pfeil 61) zu erkennen.

Zusätzlich kann nun der Druck auf die Körperoberfläche verändert werden. Dadurch ist es möglich, die durch den lokal veränderten Druck verursachten Änderungen im Gewebe wie auch z.B. das Zusammendrücken von Adern zu beobachten.

Wird dabei vor Beginn der Messung zunächst die Messanordnung an die Körperoberfläche ohne Druck aufgesetzt, so kann in diesem Zustand ein Nullabgleich vorgenommen werden und danach der Anpressdruck absolut gemessen werden.

Vorteilhaft wirkt sich dabei auch das starre Gehäuse der Messkapsel aus, die ein Nachgeben und damit Verformen der flexiblen Membran bei veränderlichen Anpressdrücken verringert.

Die Erfinder sehen insbesondere auch eine Anwendungsmöglichkeit in der Ultraschallausbildung in der medizinischen Diagnostik und evtl. auch in dem Ersatz von teuren zusätzlichen Apparaturen oder invasiven Untersuchungen in der Medizin (z.B. invasive blutige Druckmessung in Gefässen). Auch für Messungen an Tieren ist die Erfindung einsetzbar.

Für das Gehäuse der Druckmesseinrichtung sind verschiedene Materialien einsetzbar, z.B. schalldurchlässiger Plastikguss allein oder in Kombination mit verstärkenden Metallteilen.

Der Effekt der durch den verschiedenen Auflagedruck erzeugten Gewebe-/Gefässveränderung hängt von der Auflagefläche des Messkopfes und der Druckmesseinrichtung ab. Je nach Fragestellung können dieser in verschiedener Form und Grösse gebaut werden.

Auf Grund der dargestellten Beschreibung sind dem Fachmann eine Vielzahl von Abwandlungen zugänglich, ohne den Bereich der Erfindung wie in den Ansprüchen definiert zu verlassen. Denkbar sind z.B. folgende Abwandlungen:
1. Anstelle der flüssigkeitsgefüllten Messkammer wird ein elastisches, schalldurchlässiges Material eingesetzt und die Verformung gemessen.
2. Anstelle der Druckmessung in der unter Druck gesetzten Flüssigkeit wird die Verformung der Auflagemembran gemessen, z.B. durch Druckmessstreifen.
3. Eine Druckmessung kann auch über eine einfache Wassersäule, die in Verbindung mit der Flüssigkeit im Druckmesser steht, erfolgen.

## Patentansprüche

1. Druckmesseinrichtung (5) für Ultraschallvorrichtungen (7) zur Durchführung von Ultraschallmessungen, insbesondere an Lebewesen und/oder Menschen, mit räumlicher Auflösung des Messresultats in wenigstens einer Dimension, wobei die Druckmesseinrichtung (5) der Messung des Auflagedrucks der Ultraschallmessvorrichtung dient, **dadurch gekennzeichnet, dass** die Druckmesseinrichtung ein Gehäuse mit einer Vorderseite und einer gegenüberliegenden Rückseite umfasst, in die Rückseite ein Ultraschall-durchlässiges, im Wesentlichen starres Fenster (20) eingesetzt ist, an der Vorderseite eine flexible, Ultraschall-durchlässige Membran (13) aus einem Silikonelastomer vorhanden ist, Fenster und Membran Wände des Gehäuses darstellen, das Gehäuse und das Fenster bei den zu messenden Drücken im Wesentlichen starr sind, und die Druckmesseinrichtung mit einer Ultraschalldurchlässigen Flüssigkeit gefüllt ist, so dass durch die Druckmesseinrichtung hindurch mittels einer an das Fenster angekoppelten Ultraschall-Messvorrichtung Ultraschallmessungen möglich sind.

2. Druckmesseinrichtung gemäss Anspruch 1, **dadurch gekennzeichnet, dass** das Fenster (20) aus einem fluorierten Ethylenpolymer, insbesondere Poly-Tetrafluorethylen besteht.

3. Druckmesseinrichtung (5) gemäss Anspruch 2, **dadurch gekennzeichnet, dass** das Fenster (20) im Bereich, der für die Ultraschall-Durchstrahlung vorgesehen ist, eine Dicke von höchstens 1 mm und bevorzugt von 0,5 - 0,7 mm aufweist.

4. Druckmesseinrichtung (5) gemäss einem der Ansprüche 1 bis 3, **dadurch gekennzeichnet, dass** die Membran (13) eine Dicke von 0,4 bis 0,5 mm aufweist.

5. Druckmesseinrichtung gemäss einem der Ansprüche 1 bis 4, **dadurch gekennzeichnet, dass** die Flüssigkeit (11) wasserhaltig ist.

6. Druckmesseinrichtung gemäss Anspruch 5, **dadurch gekennzeichnet, dass** die Flüssigkeit eine Glycerin-Wassermischung ist, bevorzugt in einem Massenverhältnis von etwa 85:15.

7. Druckmesseinrichtung (5) gemäss einem der Ansprüche 1 bis 6, **dadurch gekennzeichnet, dass** die Schichtdicke der Flüssigkeit wenigstens im Bereich, der für den Durchtritt von Ultraschall vorgesehen ist, höchstens 1 cm beträgt.

8. Druckmesseinrichtung (5) gemäss einem der Ansprüche 1 bis 7, **dadurch gekennzeichnet, dass** sie mit einem Druckmessgerät verbunden ist, um den Druck auf die Membran (13) durch Messung des Drucks in der Flüssigkeit (11) bestimmen zu können.

9. Ultraschallmessvorrichtung (7) mit einer an die Messfläche (22) angekoppelten Druckmesseinrichtung (5) gemäss einem der Ansprüche 1 bis 8, wobei das Fenster (20) der Druckmesseinrichtung an der Messfläche der Ultraschallvorrichtung anliegt, bevorzugt mit einem Gel (8) zur Verbesserung der Kopplung.

10. Ultraschallmessvorrichtung (7) gemäss Anspruch 8, **dadurch gekennzeichnet, dass** die Druckmesseinrichtung (5) einen integrierten Bestandteil der Ultraschallmessvorrichtung bildet.

11. Verwendung einer Ultraschallmessvorrichtung (5,7) gemäss einem der Ansprüche 9 oder 10 oder einer Druckmesseinrichtung (5) gemäss einem der Ansprüche 1 bis 8 zur Durchführung von Ultraschallmessungen an Lebewesen mit dem Auflagedruck der Ultraschallmessvorrichtung als zusätzlichen Auswertungsparameter.

12. Verwendung einer Ultraschallmessvorrichtung (5,7) gemäss einem der Ansprüche 9 oder 10 oder einer Druckmesseinrichtung (5) gemäss einem der Ansprüche 1 bis 8 zur Ausbildung von Personen zur Durchführung von Ultraschallmessungen, um die Druckausübung während einer Messung trainieren und angleichen zu können.
